# EUROPÄISCHE PATENTSCHRIFT

(11) **EP 0 405 043 B1**
(45) Veröffentlichungstag und Bekanntmachung des Hinweises auf die Patenterteilung: **25.05.1994**
(21) Anmeldenummer: 89810142.3
(22) Anmeldetag: 22.02.1989
(51) Int. Cl.: A61F 13/20, A61F 13/00

(54) **Scheibe zum Auftragen u./oder Aufsaugen von flüssigen oder halbfesten Substanzen**
Pad for applying and/or removing fluid or semi-fluid materials
Tampon pour appliquer et/ou ôter des substances liquides ou semi-fluides

(43) Veröffentlichungstag der Anmeldung: 02.01.1991
(73) Patentinhaber: Flawa Schweizer Verbandstoff- und Wattefabriken AG, CH-9230 Flawil (CH)
(72) Erfinder: Gerhartl, Gerd W.P., CH-9248 Bichwil (CH); Hösli, Georg, CH-9230 Flawil (CH); Werner, Ernst, CH-9230 Flawil (CH)
(74) Vertreter: Kulhavy, Sava, Dipl.-Ing.

(56) Entgegenhaltungen:
- DE-A- 3 341 143
- FR-A- 2 052 089
- FR-A- 2 502 470
- GB-A- 2 024 709
- US-A- 3 055 035

## Beschreibung

Die vorliegende Erfindung betrifft eine Scheibe zum Auftragen und/oder Aufsaugen von flüssigen oder halbfesten Substanzen, mit zumindest drei aufeinander liegenden Schichten.

Ein Gegenstand dieser Gattung ist aus GB-A 2 024 709 bekannt. Bei diesem Gegenstand handelt es sich um ein flächenhaftes Gebilde, welches drei aufeinander liegende Schichten aus Fasern enthält. Die sich an die beiden Seiten der mittleren Schicht anschliessenden Schichten sind dünner als die mittlere Schicht. Nachdem dieses flächenhafte Gebilde entstand, wird es zwischen zwei Walzen geführt und dabei lokal zusammengepresst. Die Aussenseite der jeweiligen Walze ist mit schraubenlienförmig verlaufenden Rillen versehen, zwischen welchen sich hervortretende Partien dieser Walzen befinden. Die Schraubenlinien der Walzen sind gegenläufig. Wenn das textile Gebilde zwischen solchen Walzen geführt wird, entstehen in diesem zusammengepresste Bereiche, welche durch die Einwirkung der hervortretenden Partien der Walzen auf die Warenbahn verursacht sind und welche sich schräg zur Längsrichtung der Warenbahn erstrecken. Da die schraubenlinienförmigen Vorsprünge gegenläufig sind, verlaufen die zusammengepressten linienförmigen Bereiche der Warenbahn schräg zueinander, sodass sich diese Bereiche auch kreuzen. An jenen Stellen, wo sich die Vorsprünge der Walzen gekreuzt haben, ist das Material der Warenbahn besonders stark zusammengepresst.

Eine solche Warenbahn ist zum Auftragen oder/und Aufsaugen von flüssigen, pastösen oder ähnlichen Mitteln nicht geeignet, weil das Material derselben, insbesondere dort, woch sich die stark zusammengepressten Bereiche der Warenbahn befinden, praktisch keine Saugfähigkeit aufweist.

Eine Scheibe der eingangs genannten Gattung ist auch in FR-A-2 052 089 offenbart. Diese vorbekannte Scheibe umfasst zwei aufeinander liegende Schichten aus Watte oder aus Baumwolle auf. Diese Schichten sind über ihre Grossflächen miteinander verbunden. Die flächemässige Verbindung der Materialschichten kann durch Vernadelungsstellen erreicht werden, welche über die ganze Grossfläche der Scheiben verteilt sind. Die genannte Verbindung kann jedoch auch durch Zusammenpressen des Materials der beiden Lagen entlang geraden Linien erfolgen, welche parallel zueinander verlaufen und welche sich über die ganze Länge der Grossfläche der Schichten erstrecken. Die Randpartien der Schichten bleiben dabei frei.

Die Aufgabe, welche n.a. die Massnahme nach diesem zuletzt genannten Dokument des Standes der Technik lösen soll, ist, zu verhindern, dass die Scheiben, wenn sie sich in Form eines Stapels in einer Vorratspackung befinden, aufeinander haften. Dies soll sicherstellen, dass jeweils nur eine einzige dieser Scheiben der Packung entnommen werden kann. Die Vernadelungsstellen und insbesondere die Drucklinien im mittleren Bereich der Scheibe beeinträchtigen die Saugfähigkeit dieser vorbekannten Scheiben sehr stark. Ausserdem kann die Berührung der Haut, beispielsweise im Gesicht, durch die versteiften Stellen der Oberfläche der vorbekannten Scheibe unangenehme Empfindung beim Benützer bzw. bei der Benützerin der vorbekannten Scheibe hervorrufen. Ausserdem besteht die nachteilige Möglichkeit, dass das Scheibenmaterial aus den Randbereichen der vorbekannten Scheibe bei der Benützung derselben herausgerissen und auf der Haut haften bleibt.

Die Aufgabe der vorliegenden Erfindung ist die genannten und noch weitere Nachteile der vorbekannten Scheiben zu beheben.

Diese Aufgabe wird bei der Scheibe der eingangs genannten Gattung erfindungsgemäss so gelöst, wie dies im kennzeichnenden Teil des Anspruchs 1 definiert ist.

Nachstehend werden Ausführungsbeispiele der vorliegenden Erfindung anhand der beiliegenden Zeichnung näher erläutert. Es zeigt:
Fig. 1 perspektivisch die vorliegende Scheibe, bei welcher die Schichten, welche sie aufweist und deren Ränder normalerweise untereinander verbunden sind, in einem Bereich derselben in Abstand voneinander gebracht worden sind,
Fig. 2 perspektivisch eine erste Art von Vertiefungen in einer der Scheibenschichten, welche einen linienförmigen Verlauf aufweisen, und
Fig. 3 perspektivisch eine zweite Art der genannten Vertiefungen, welche ein punktförmiges Muster bilden.

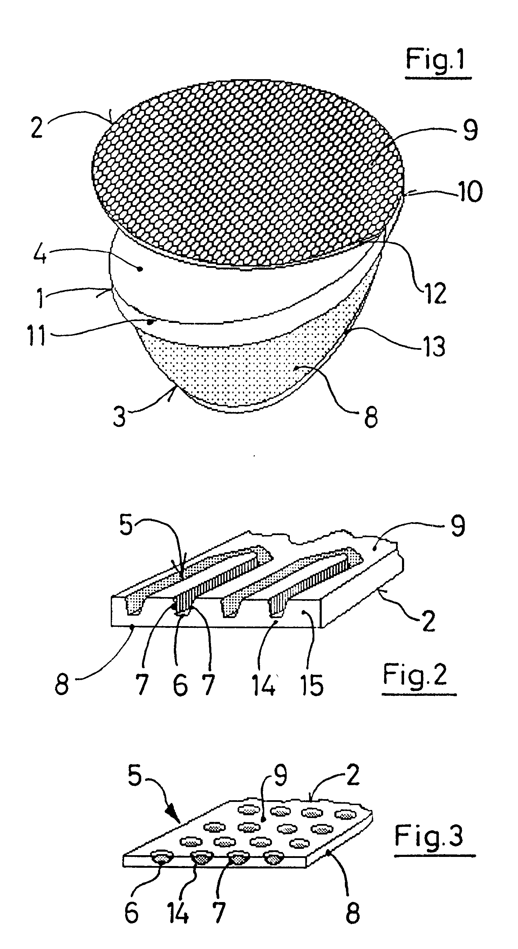

Die in Fig. 1 perspektivisch dargestellte Scheibe kann beispielsweise zum Auftragen, Abtragen und/oder Aufsaugen von flüssigen oder halbfesten Substanzen dienen. Sie weist in diesem dargestellten Beispiel drei Schichten bzw. Lagen auf, von welchen jede scheibenförmig ist. In Fig. 1 ist die vorliegende Scheibe so dargestellt, dass deren Ränder, die normalerweise untereinander verbunden sind, sich in einem Bereich derselben in einem Abstand voneinander befinden. Der mittleren Schicht 1 der Scheibe sind zwei weitere, äussere Schichten 2 und 3 zugeordnet, und zwar derart, dass die äusseren Schichten 2 und 3 auf den planen Oberflächen 4 der mittleren Schicht 1 aufliegen.

Die Schichten 1 bis 3 können aus demselben Material oder aus unterschiedlichen Materialen sein. Das Material dieser Schichten 1 bis 3 soll saugfähig sein. Vorteilhaft ist dieses Material bzw. sind die erwähnten Materialien faserig, wobei das Fasermaterial Baumwolle, Viscose, synthetische Fasern oder ein Gemischt aus diesen sein kann. Besonders vorteilhaft ist es, wenn für die Schichten 1 bis 3 Watte aus Baumwolle verwendet wird.

Die Materialschichten 1, 2 und 3 sind einander derart zugeordnet, dass sie eine zusammenhaltende Sandwich-Struktur bilden. Dies wird beispielsweise dadurch erreicht, dass die Randpartien 11, 12 und 13 der genannten Materialschichten 1 bis 3 untereinander verbunden sind. Es liegen die Ränder 11 bis 13 aller drei Schichten 1 bis 3 aufeinander (Fig. 1) und die Ränder 11 bis 13 aller drei Schichten 1 bis 3 werden miteinander verbunden. Bei einer zweiten Art der Verbindung hat die mittlere Schicht 1 einen kleineren Durchmesser als die Aussenschichten 2 und 3, so dass der Rand 11 der Innenschicht 1 sich im Inneren der Sandwich-Struktur, d.h. in einem Abstand von den Rändern 12 und 13 der äusseren Schichten 2 und 3, befindet (nicht dargestellt). Bei dieser zweiten Verbindungsart werden nur die Ränder 12 und 13 der Aussenschichten 2 und 3 miteinander verbunden, so dass die mittlere Schicht 1 samt ihrer Randpartie 11 zwischen diesen eingeschlossen ist. Die Verbindung der Randpartien 11 bis 13 untereinander kann während der Herstellung der Scheibe erreicht werden. Zu diesem Zweck muss man allerdings geeignete Stanzmesser benützen. Nach einem solchen Schnitt ist das Produkt rund herum verschlossen. Andererseits könnte man auch geeignete Klebemittel zur Verbindung der genannten Ränder verwenden.

Es ist möglich, den Zusammenhalt zwischen den Schichten 1 bis 3 dadurch zu verbessern, dass das fertige Produkt zusammengepresst wird, wodurch eine flächenhafte Verbindung zwischen den Materialen an der Innenseite 8 der Aussenschichten 2 bzw. 3 und an den planen Oberflächen 4 der Mittelschicht 1 erreicht wird.

Das Material der äusseren Schichten 2 und 3 ist durch ein erstes und flächenhaftes, d.h. über die ganze Fläche der Schicht 2 bzw. 3 gleichmässiges Zusammenpressen desselben verdichtet. Das Material einer solchen Schicht 2 bzw. 3 ist verfestigt und die Oberfläche desselben ist praktisch faserfrei. Eine solche Schicht ist gut flüssigkeitsdurchlässig aber sie ist nicht sehr saugfähig. Das Material der inneren Schicht 1 weist dagegen keine oder wenigstens keine nennenswerte Verdichtung auf. Deswegen bildet die innere bzw. mittlere Schicht 1 den eigentlichen saugfähigen Grundkörper der vorliegenden Scheibe. Die Aussenschicht 2 bzw. 3 kann beispielsweise eine Dicke von etwa 0,8 mm und die Innenschicht 1 kann eine Dicke von etwa 1,5 mm aufweisen.

Die Aussenschicht 2 bzw. 3 kann mit einem Muster versehen sein, welches durchgehende Oeffnungen (nicht dargestellt) in der Aussenschicht 2 bzw. 3 oder Vertiefungen 5 in dieser aufweist. Die Oeffnungen in der Aussenschicht können durch Stanzen der bereits flächenhaft verdichteten Materialschicht hergestellt werden. Zu diesem Zweck kann man die bereits erwähnten speziellen Stanzmesser verwenden, damit die Kanten dieser Oeffnungen geschlossen und somit nicht fasernd sind.

Die Vertiefungen 5 werden durch eine zusätzliche und nur örtlich wirksame Verdichtung des Materials der Aussenschicht 2 bzw. 3 hergestellt. Diese zusätzlich verdichteten Stellen 5 werden als senkrecht zur Oberfläche 9 der Aussenschicht 2 bzw. 3, beispielsweise durch Prägung mit Hilfe von profilierten Walzen, zusammengepresste Bereiche ausgeführt. In Fig. 2 ist eine linienförmige Prägung 5 und in Fig. 3 ist eine punktförmige Prägung 5 der Aussenschicht 2 bzw. 3 dargestellt.

Die Vertiefung 5 in der Aussenschicht 2 bzw. 3 (Fig. 2) weist einen Boden 6 auf, welcher unterhalb der Oberfläche 9 der weniger, d.h. nur primär verdichteten Materialschicht 2 bzw. 3 liegt. Seitlich schliessen sich Seitenwände 7 an den Boden 6 der linienförmigen Vertiefung 5 an, welche in der Oberfläche bzw. Aussenfläche 9 der nur primär und flächenhaft verdichteten Aussenschicht 2 bzw. 3 enden. Die zusätzliche Verdichtung 5 wird so durchgeführt, dass die Dicke des Schichtabschnittes 14, der sich zwischen dem Boden 6 der Vertiefung 5 und der Unterseite 8 der Aussenschicht 2 bzw. 3 befindet, maximal die Hälfte der Dicke des ungeprägten und somit gegenüber dem Boden 6 erhabenen Bereiches 15 der Aussenschicht 2 bzw. 3 ausmacht, d.h. unter Umständen kann die zusätzliche Verdichtung so weit gehen, dass die Dicke des Schichtabschnittes 14 noch kleiner ist als die Hälfte der Dicke der Aussenschicht 2 bzw. 3.

Wie aus Fig. 2 ersichtlich ist, bilden die einzelnen Vertiefungen 5 linienförmige Kanäle, welche parallel nebeneinander verlaufen und untereinander verbunden sind. Diese Kanäle 5 sind verhältnismässig breit und lang, so dass sie viel pastöses bis fliessfähiges Material aufnehmen können. Das Material der Kanalflanken 7 ist weniger verpresst als das Material 14 unter dem Boden 6, so dass die Flanken 7 Flüssigkeiten besser aufnehmen können. Der Hohlraum der Vertiefungen 5 des Prägemusters ermöglicht, wie gesagt, die Aufnahme von halbfesten Substanzen und man kann diese Hohlräume 5 als Depot für die Substanzen bezeichnen. Die erhabenen, d.h. weniger verdichteten Stellen 15 wirken wie ein Saugkissen. Eine Aussenschicht 2 bzw. 3 mit einem solchen Muster 5 kann somit zum Abtragen einer bereits aufgetragenen Creme oder zu ähnlichen Zwecken hervorragend verwendet werden.

Das Tiefenmuster gemäss Fig. 3 weist punktförmige Vertiefungen 5 auf, deren Wand 7 die Form einer Hälfte von Kugelmantel aufweist. Es gibt eine grosse Anzahl solcher Vertiefungen 5 in der Oberfläche 9 der Aussenschicht 2 bzw. 3 und zudem ist diese Schicht sehr stark zusätzlich verdichtet. Denn die sich unter der Vertiefung 5 befindliche Materialschicht 14 ist sehr dünn, wobei auch die Dicke dieser Aussenschicht kleiner ist als die Dicke der Aussenschicht gemäss Fig. 2. Das Material dieser so dünnen Aussenschicht 2 bzw. 3 ist sehr dicht und folglich ist es auch wenig saugfähig. Eine solche Aussenschicht eignet sich daher hervorragend zum Auftragen von Kosmetika auf die Haut oder ähnlich, weil sie nur wenig von einer solchen Substanz in sich aufnehmen kann.

Die Tiefenmuster 5 sind an wenigstens einer der Seiten 8 oder 9 der Aussenschicht 2 bzw. 3 ausgeführt. Die Aussenschicht 2 bzw. 3 ist auf der Innenschicht 1 so angebracht, dass die mit dem Muster 5 versehene Seite 9 der Aussenschicht 2 bzw. 3 an der Aussenseite der Scheibe liegt. Die Muster 5 in den äusseren Seiten 9 der Aussenschichten 2 und 3, welche auf einer mittleren Schicht 1 angebracht sind, können gleich oder unterschiedlich ausgebildet sein. Zu diesem Zweck sind die Tiefe und/oder die Fläche der Vertiefungen in den Aussenschichten 2 und 3 unterschiedlich gross. Folglich weist eine solche Scheibe unterschiedlich stark verdichtete Aussen- bzw. Oberflächen und somit auch unterschiedliche Eigenschaften auf.

Solche Muster 5 können auch in den beiden Seiten 8 und 9 der jeweiligen Aussenschicht 2 bzw. 3 ausgeführt sein. Dann kann es vorteilhaft sein, dass die Vertiefungen in den gegenüberliegenden Seiten 8 und 9 einer Aussenschicht 2 bzw. 3 zueinander versetzt ausgeführt sind. Dies bedeutet, dass der Boden 6 der Vertiefung 5 in der einen Seite 8 zwischen zwei Vertiefungen 5 in der gegenüberliegenden Seite 9 liegt (nicht dargestellt).

Durch die Grösse der ersten bzw. primären Verdichtung sowie der zusätzlichen bzw. sekundären Verdichtung des Materials der Aussenschichten 2 und 3 kann man die physikalischen Eigenschaften dieser Schichten wählen und steuern. Diese können auch durch die Art, das Muster, die Tiefe, die Grösse der Fläche und Dichte der Prägung sowie durch die Form des Querschnittes der Vertiefungen 5 gesteuert werden.

Eine Watterondelle für kosmetische Zwecke, welche eine Dicke von etwa 3,7 mm aufweist, besteht z.B. aus einem unverdichteten Mittelteil 1 von etwa 1,5 mm Dicke und je einer verdichteten oberen und unteren Aussenschicht 2 und 3 von je etwa 0,8 mm Dicke. Die Oberflächen der Scheibe sind beide mit einem Waffelmuster versehen, die eine mit einem sehr engen, die andere mit einem breiten. Das Produkt fühlt sich sehr weich und flauschig an, fasert und staubt nicht und weint an der grobgemusterten, weniger verdichteten Seite ein ausgezeichnetes Saugvermögen auf.

Das Verdichten des Materials der Aussenschichten 2 und 3 erfolgt im allgemeinen durch Kalandrieren. Das zusätzliche Verdichten der Aussenschichten 2 und 3, während welchem Hohlräume 5 in der Aussenschicht 2 bzw. 3 entstehen, erfolgt ebenfalls durch Kalandrieren und es werden, je nach Bedarf, engere oder weitere Prägemuster an den Walzen verwendet. Je nach Material erfolgt die Kalandrierung vorzugsweise bei 100 bis 200 Grad C mit einem Druck von bis zu 0,5 kg/cm2 und bei einer Walzengeschwindigkeit von 5 bis 25 m/min. Wenn bis zu 10% der Oberfläche der Aussenschicht 2 bzw. 3 geprägt sind, entsteht ein saugfähiges Material, das sich zur Aufnahme von Flüssigkeiten eignet und nicht fasert. Mit steigendem Anteil an tiefgeprägter Fläche wird das Material der Aussenschichten 2 und 3 weniger saugfähig.

Das Verdichten kann auch mit Hilfe chemischer Substanzen erfolgen, z.B. physiologisch unbedenklicher, hautfreundlicher, luft- und feuchtigkeitsdurchlässiger Kunstharze, mit welchen die Oberfläche je nach Bedarf mehr oder weniger stark behandelt wird. Solche Harze sind dem Fachmann für die Herstellung verschiedener Watte- und ähnlicher Artikel bekannt.

Die Faseraufbereitung zum Faserflor erfolgt auf übliche Weise, z.B. auf Karden, Krempeln oder ähnlichen geeigneten Maschinen. Daraufhin wird der Faserflor zu mindestens drei kontinuierlichen Vliesbahnen bzw. -flächen mit dem gewünschten Gesamtgewicht je Längeneinheit zusammengefasst, worauf das Prägen der äusseren, d.h. der ersten und dritten Vliesflächen mit den gewünschten und für das Anwendungsprofil erforderlichen Mustern 5 auf bekannte Weise durchgeführt wird. Die drei und gegebenenfalls mehr Vliesbahnen werden sodann zu einem Aufbau geprägt / lose bzw. ungeprägt, zusammengeführt. Der derart erhaltene bahnförmige Aufbau wird nun in die gewünschte Form gestanzt oder geschnitten und auf geeignete Weise verpackt. Je nach Verwendungszweck kann zusätzlich vor oder nach dem Zerkleinern eine Weiterbehandlung, wie Keimfreimachen, mit Wirkstoffen beladen, usw., erfolgen. Dieses Verfahren erlaubt es, übliche Vorrichtungen zu verwenden, wobei Produkte mit glatter, faserfreier, nichtstaubender, aber doch weicher Oberfläche und einem sauberen Randverschluss entstehen. Die Herstellung kann jedoch auch mit bekannten Maschinen auf aerodynamischem Wege erfolgen.

Im einzelnen kann die Herstellung der vorliegenden Scheiben wie folgt beschrieben werden:
100%ige Baumwollkämmlinge, rein, weis gebleicht, werden auf einer Karde zum Faserflor aufbereitet. Dieser Faserflor wird zu drei kontinuierlichen Vliesbahnen mit einem Gesamtgewicht von etwa 350 g/m2 zusammengefasst. Zwei dieser Vliesflächen werden getrennt in einem Prägekalander flächenhaft verdichtet und mit einem Waffelmuster versehen, wobei diese Bemusterung 5 die örtliche zusätzliche Verdichtung des Fasermaterials zur Folge hat. Dieses Prägen erfolgt bei einer Walzentemperatur von 150 Grad C, einer Durchlaufgeschwindigkeit von 12 m/min. und einem Druck von 1100kg/20 cm linear. Die drei Vliesflächen werden dann derart zu einem sandwichartigen Aufbau zusammengeführt, dass die beiden verdichteten und zusätzlich geprägten Bahnen die Aussenseite 2 bzw. 3 bilden und dass die dritte, praktisch unverdichtete Fläche zwischen diese beiden Aussenschichten zu liegen kommt. Diese bildet dann den saugfähigen Kern 1 der Scheibe. Diese Materialbahn läuft nunmehr unter eine Stanze, aus der die gewünschten Rondellen ausgestanzt werden. Diese werden aufeinandergelegt und in Plastikbeutel zu einer Rolle verpackt.

Die vorliegenden Scheiben, sind insbesondere zum Auftragen und/oder Aufsaugen von flüssigen oder halbfesten Substanzen, wie von kosmetischen, pharmazeutischen und biologischen Flüssigkeiten, Salben, Exudaten, usw. ebenso wie technischen Substanzen jeder Art verwendbar. Die Verdichtung 5 der Aussenschichten 2 und 3 verhindert weitgehend das sogenannte Fuseln bzw. Fasern, d.h. das unerwünschte Austreten einzelner Fasern aus der Wattescheibe und Klebenbleiben dieser auf der Haut oder einer anderen Fläche. Die Verdichtung 5 verhindert auch das sogenannte "Stäuben".

Mit einer solchen Scheibe lässt sich beispielsweise eine Flüssigkeit oder Creme auftragen, ohne dass ein grosser Anteil davon in die Scheibe eindringt und damit für den Konsumenten verloren geht. Eine schwächere Verdichtung an der Rückseite derselben Scheibe erlaubt anderseits, durch einfaches Umdrehen der Scheibe, den Ueberschuss an aufgetragener Substanz oder aber auch z.B. eine bereits aufgetragene Reinigungscreme, abzutragen, wobei das Aufsaugen durch die weniger verdichtete und daher saugfähigere Oberfläche keinerlei Schwierigkeiten bereitet.

Die vorliegende Scheibe ist eine auf einfache und wirtschaftliche Weise herstellbare Scheibe, deren Ausbildung zudem an verschiedene Zwecke problemlos angepasst werden kann.

## Patentansprüche

1. Scheibe zum Auftragen und/oder Aufsaugen von flüssigen oder halbfesten Substanzen, mit zumindest drei aufeinander liegenden Schichten (1,2,3), dadurch gekennzeichnet, dass das Material der äusseren Schichten (2,3) durch ein gleichmäßiges Zusammenpressen über die ganzen Schichtflächen verdichtet ist, dass das Material der inneren Schicht (1) eine nur durch das Verbinden der Schichten (1,2,3) miteinander bedingte möglicherweise geringe Verdichtung aufweist und dass diese Materialschichten (1,2,3) einander derart zugeordnet sind, dass sie eine zusammenhaltende Sandwich-Struktur bilden, wobei die Randpartien (12, 13) zumindest der äusseren Materialschichten (2,3) miteinander verbunden sind.

2. Scheibe nach Anspruch 1, dadurch gekennzeichnet, dass die Randpartien (11,12,13) aller drei Schichten (1,2,3) miteinander verbunden sind oder dass die mittlere Schicht (1) einen kleineren Durchmesser aufweist als die Aussenschichten (2,3), so dass die Ränder (12,13) nur der Aussenschichten (2,3) miteinander Verbunden sind, und dass die Verbindung dieser Randpartien durch Stanzen mittels geeigneter Stanzmesser oder durch Zusammenkleben derselben mittels Klebemittel erfolgen kann.

3. Scheibe nach Anspruch 1, dadurch gekennzeichnet, dass zumindest eine der Aussenschichten (2 oder 3) mit einem Muster versehen ist, und dass dieses Muster durchgehende Oeffnungen in der Aussenschicht (2,3) oder wenigstens Vertiefungen (5) umfasst.

4. Scheibe nach Anspruch 3, dadurch gekennzeichnet, dass die jeweilige Vertiefung (5) in der Aussenschicht (2 oder 3) einen Boden (6) aufweist, welcher unterhalb der Oberfläche (9) des flächenhaft zusammengepressten Materials der Aussenschicht (2 bzw. 3) liegt, dass seitlich sich Seitenwände (7) an den Boden (6) der Vertiefung (5) anschliessen, welche in der Oberfläche bzw. Aussenfläche (9) der Aussenschicht (2 bzw. 3) enden, und dass die Dicke des Schichtabschnittes (14), der sich zwischen dem Boden (6) der Vertiefung (5) und der Unterseite (8) der Aussenschicht (2 bzw. 3) befindet, die Hälfte der Dicke des ungeprägten und somit gegenüber dem Boden (6) erhabenen Bereiches (15) der Aussenschicht (2 bzw. 3) ausmachen oder noch kleiner sein kann.

5. Scheibe nach Anspruch 4, dadurch gekennzeichnet, dass die Vertiefungen (5) linienförmige Kanäle bilden, welche parallel nebeneinander verlaufen und untereinander verbunden sind, oder dass das Tiefenmuster punktförmige Vertiefungen (5) aufweist, deren Wand (7) die Form einer Hälfte von Kugelmantel aufweist.

6. Scheibe nach Anspruch 3, dadurch gekennzeichnet, dass das Tiefenmuster (5) an wenigstens einer der Seiten (8 oder 9), vorteilhaft an der Aussenseite (9), der Aussenschicht (2 bzw. 3) ausgeführt ist, und dass die Aussenschicht (2 bzw. 3) auf der Innenschicht (1) so angebracht ist, dass die mit dem Muster (5) versehene Seite (9) der Aussenschicht (2 bzw. 3) an der Aussenseite der Scheibe liegt.

7. Scheibe nach Anspruch 6, dadurch gekennzeichnet, dass die Muster (5) in den beiden Seiten (8 und 9) der jeweiligen Aussenschicht (2 bzw. 3) ausgeführt sind und dass die Vertiefungen in den gegenüberliegenden Seiten (8 und 9) derselben Aussenschicht (2 bzw. 3) zueinander versetzt angeordnet sind.

8. Scheibe nach Patentanspruch 6, dadurch gekennzeichnet, dass die Tiefe und/oder die Fläche der Vertiefungen (5) in den Aussenschichten (2 und 3), die auf den gegenüberliegenden Seiten (4) der Innenschicht (1) angebracht sind, unterschiedlich gross sind, so dass die Scheibe unterschiedlich stark verdichtete Oberflächen aufweist.

9. Scheibe nach Patentanspruch 1, dadurch gekennzeichnet, dass wenigstens eine der Schichten ein Material enthält, welches eine kosmetische oder/und heilende Wirkung zeigen kann.

## Claims

1. Pad for applying and/or removing fluid or semi-fluid materials, having at least three plies (1,2,3) lying one on the other, characterized in that the material of the outer plies (2,3) is compacted as a result of a uniform compression over the entire ply surfaces, in that the material of the inner ply (1) has a possibly low compaction caused only by the interconnection of the plies (1,2,3), and in that these material plies (1,2,3) are assigned to one another in such a way that they form a coherent sandwich structure, the edge parts (12,13) at least of the outer material plies (2,3) being connected to one another.

2. Pad according to Claim 1, characterized in that the edge parts (11,12,13) of all three plies (1,2,3) are connected to one another, or in that the middle ply (1) has a smaller diameter than the outer plies (2,3), so that the edges (12,13) of only the outer plies (2,3) are connected to one another, and in that the connection of these edge parts can take place by punching by means of suitable punching knives or by the bonding together of these by means of adhesive.

3. Pad according to Claim 1, characterized in that at least one of the outer plies (2 or 3) is provided with a pattern, and in that this pattern comprises continuous orifices in the outer ply (2,3) or at least depressions (5).

4. Pad according to Claim 3, characterized in that the respective depression (5) in the outer ply (2 or 3) has a bottom (6) which is located underneath the surface (9) of the superficially compressed material of the outer ply (2 or 3), in that side walls (7) laterally adjoin the bottom (6) of the depression (5) and terminate in the surface or outer face (9) of the outer ply (2 or 3), and in that the thickness of the ply portion (14) located between the bottom (6) of the depression (5) and the underside (8) of the outer ply (2 or 3) can amount to or even be smaller than half the thickness of the region (15) of the outer ply (2 or 3) which is unembossed and therefore raised relative to the bottom (6).

5. Pad according to Claim 4, characterized in that the depressions (5) form linear channels which extend next to one another in parallel and which are connected to one another, or in that the depth pattern has punctiform depressions (5), the wall (7) of which has the form of one half of a ball envelope.

6. Pad according to Claim 3, characterized in that the depth pattern (5) is formed on at least one of the sides (8 or 9), advantageously on the outer side (9), of the outer ply (2 or 3), and in that the outer ply (2 or 3) is attached to the inner ply (1) in such a way that the side (9) of the outer ply (2 or 3) provided with the pattern (5) is located on the outer side of the pad.

7. Pad according to Claim 6, characterized in that the patterns (5) are formed in the two sides (8 and 9) of the respective outer ply (2 or 3), and in that the depressions are arranged offset relative to one another in the opposite sides (8 and 9) of the same outer ply (2 or 3).

8. Pad according to Patent Claim 6, characterized in that the depth and/or the area of the depressions (5) in the outer plies (2 and 3) which are attached to the opposite sides (4) of the inner ply (1) are of differing amount, so that the pad has surfaces compacted to a differing extent.

9. Pad according to Patent Claim 1, characterized in that at least one of the plies contains a material which can have a cosmetic and/or healing effect.

## Revendications

1. Tampon pour appliquer et/ou enlever des substances liquides ou semi-solides, avec au moins trois couches superposées (1, 2, 3), caractérisé en ce que la matière des couches externes (2, 3) est comprimée par un serrage uniforme sur les faces entières des couches, en ce que la matière de la couche interne (1) présente une compression aussi faible que possible imposée uniquement par l'assemblage des couches (1, 2, 3) et en ce que ces couches (1, 2, 3) sont agencées l'une par rapport à l'autre de telle sorte qu'elles forment une structure sandwich cohérente, dans laquelle les zones de bord (13) au moins des couches externes (2, 3) sont assemblées l'une à l'autre.

2. Tampon suivant la revendication 1, caractérisé en ce que les zones de bord (11, 12, 13) des trois couches (1, 2, 3) sont assemblées l'une à l'autre ou en ce que la couche centrale (1) présente un plus petit diamètre que les couches externes (2, 3), de telle sorte que les bords (12, 13) des seules couches externes (2, 3) soient assemblés l'un à l'autre, et en ce que l'assemblage de ces zones de bord peut être réalisé par poinçonnage au moyen d'outils de poinçonnage appropriés ou par collage de ces dernières au moyen d'un adhésif.

3. Tampon suivant la revendication 1, caractérisé en ce qu'au moins une des couches externes (2 ou 3) est pourvue d'un motif, et en ce que ce motif comprend des trous traversants dans la couche externe (2, 3) ou au moins des empreintes (5).

4. Tampon suivant la revendication 3, caractérisé en ce que l'empreinte respective (5) dans la couche externe (2 ou 3) présente un fond (6), qui est situé sous la surface (9) de la matière superficiellement comprimée de la couche externe (2, resp. 3), en ce qu'au fond (6) de l'empreinte (5) se raccordent latéralement des flancs (7), qui se terminent dans la surface respectivement dans la face extérieure (9) de la couche externe (2, resp. 3) et en ce que l'épaisseur de la portion de couche (14) qui se trouve entre le fond (6) de l'empreinte (5) et la face inférieure (8) de la couche externe (2, resp. 3), peut être égale ou même inférieure à la moitié de l'épaisseur de la zone (15) de la couche externe (2, resp. 3) non repoussée et se trouvant ainsi en relief par rapport au fond (6).

5. Tampon suivant la revendication 4, caractérisé en ce que les empreintes (5) forment des canaux linéaires, qui sont disposés parallèlement l'un à l'autre et qui sont raccordés l'un à l'autre, ou en ce que le motif en relief présente des empreintes ponctuelles (5), dont le flanc (7) présente une forme hémisphérique.

6. Tampon suivant la revendication 3, caractérisé en ce que le motif en relief (5) est réalisé dans au moins une des faces (8 ou 9), avantageusement dans la face extérieure (9), de la couche externe (2, resp. 3), et en ce que la couche externe (2, resp. 3) est posée sur la couche interne (1) de telle sorte que la face (9) de la couche externe (2, resp. 3) pourvue du motif (5) se trouve dans la face extérieure du tampon.

7. Tampon suivant la revendication 6, caractérisé en ce que les motifs (5) sont réalisés dans les deux faces (8 et 9) de chaque couche externe (2, resp. 3) et en ce que les empreintes sont disposées, en position relative décalée, dans les faces opposées (8 et 9) de la même couche externe (2, resp. 3).

8. Tampon suivant la revendication 6, caractérisé en ce que la profondeur et/ou l'étendue des empreintes (5) dans les couches externes (2 et 3), qui sont appliquées sur les faces opposées (4) de la couche interne (1), sont de taille différente, de telle sorte que le tampon présente des surfaces comprimées à des degrés différents.

9. Tampon suivant la revendication 1, caractérisé en ce qu'au moins une des couches contient une matière qui peut présenter une action cosmétique et/ou curative.
